# DEMANDE DE BREVET EUROPEEN

(11) **EP 1 118 899 A1**
(43) Date de publication de la demande: **25.07.2001**
(21) Numéro de dépôt: 00420012.7
(22) Date de dépôt: 20.01.2000
(51) Int. Cl.: G02F 1/133, G02C 7/10, A61F 9/06

(54) **Dispositif optique à filtre électro-optique commandé automatiquement en fonction de la lumière qui l'atteint**

(71) Demandeur: Dumas, Jean-Claude, 42170 Saint Just Saint Rambert (FR); Sassi, Hocine, 42160 Bonson (FR); Reymond, Jacques, 42000 Saint Etienne (FR); Sotton, André, 42120 Le Côteau (FR)
(72) Inventeur: Dumas, Jean-Claude, 42170 Saint Just Saint Rambert (FR); Sassi, Hocine, 42160 Bonson (FR); Reymond, Jacques, 42000 Saint Etienne (FR); Sotton, André, 42120 Le Côteau (FR)
(74) Mandataire: Thivillier, Patrick

(57) **Abrégé**

Ce dispositif comprend
- un capteur photosensible,
- un circuit électronique électriquement relié au capteur photosensible,
- et au moins un écran électro-optique fonctionnant en positif et comprenant une zone active et reliée par des liaisons électriques à la sortie du circuit électronique,
remarquable en ce que :
- en ce que le capteur photo-sensible est adapté à émettre un signal de puissance croissante, en fonction de l'intensité lumineuse qui l'atteint,
- et en ce que le circuit électronique, d'une part, émet un signal secondaire alternatif de puissance croissante avec la puissance du signal émis par le capteur photo-sensible et, d'autre part, présente des moyens aptes à faire varier la fréquence du signal secondaire, l'augmentation de l'intensité lumineuse se traduisant par une réduction de la fréquence .

## Description

La présente invention se rapporte à un dispositif optique à filtre électro-optique commandé automatiquement en fonction de la lumière qui l'atteint.

Elle concerne également l'utilisation dudit dispositif pour les caméras électroniques, les lunettes solaires et anti-brouillard, les lunettes de visée et viseurs pour le tir, visières pour casques pour différents sports (VTT, moto, ski, ailes volantes), vitrages véhicules tous types, les lunettes de soudage, les verres de jumelles, les filtres pour appareillage photo, les optiques de phares de voitures et les dispositifs de filtrage d'éclairage lumineux.

Les filtres connus à ce jour réalisés chimiquement ou mécaniquement accomplissent un filtrage prédéterminé et invariable en fonction de la lumière qui les atteint.

On a par ailleurs proposé dans le document FR-A-2 693 562 un dispositif possédant un capteur photosensible commandant la transparence d'écrans électro-optiques en fonction décroissante de l'intensité lumineuse qu'il capte. Plus précisément, ce dispositif présente un assemblage d'écrans à cristaux liquides et de polariseurs adapté à engendrer un contraste très uniforme dans un angle solide correspondant à la vision de l'oeil. Le mode de connexion électrique des écrans à cristaux liquides est adapté à éviter la sensation de vague dûe à l'obscurcissement progressif latéral d'un écran à cristaux liquides.

Toutefois, on obtient un obscurcissement progressif en appliquant au filtre optique un signal alternatif d'amplitude croissante ou de fréquence décroissante avec l'intensité lumineuse détectée. Malheureusement, le contraste obtenu n'est pas proportionnel avec l'intensité lumineuse de sorte que l'obscurcissement ou l'éclaircissement de l'écran apparaît pour une variation d'intensité lumineuse très faible.

L'invention s'est fixée pour but de remédier à ces inconvénients :

Le problème que se propose de résoudre l'invention est d'obtenir un contraste qui soit proportionnel à l'intensité lumineuse.

Pour résoudre le problème posé , on a conçu et mis au point un dispositif optique à filtre électro-optique comportant :
- un capteur photosensible,
- un circuit électronique électriquement relié au capteur photosensible,
- et au moins un écran électro-optique fonctionnant en positif et comprenant une zone active et reliée par des liaisons électriques à la sortie du circuit électronique,
remarquable en ce que :
- en ce que le capteur photo-sensible est adapté à émettre un signal de puissance croissante, en fonction de l'intensité lumineuse qui l'atteint,
- et en ce que le circuit électronique, d'une part, émet un signal secondaire alternatif de puissance croissante avec la puissance du signal émis par le capteur photo-sensible et, d'autre part, présente des moyens aptes à faire varier la fréquence du signal secondaire, l'augmentation de l'intensité lumineuse se traduisant par une réduction de la fréquence .

La description qui va suivre, faite dans un but explicatif et nullement limitatif, permet de mieux comprendre les avantages, buts et caractéristiques de l'invention.

La figure 1 représente une vue de face d'une paire de lunettes équipée d'un dispositif conforme à l'invention, telle que lunettes solaires, de vue, de visée pour le tir, de pêche, de sport, de soudage.

La figure 2 représente une vue en coupe d'un des écrans électro-optiques incorporés dans le dispositif représenté en figure 1.

La figure 3 représente, en vue de face et de coupe, un filtre électro-optique possédant une courbure.

La figure 4 représente un exemple de visière pour les sports tels que le ski, VTT, ailes volantes.

La figure 5 représente un exemple de filtre pouvant être installé sur un appareil photographique, viseur de carabine, caméra.

La figure 6 représente un filtre d'ambiance composé d'une association de filtres élémentaires de cristaux liquides de couleurs différentes (lampes d'ambiance).

La figure 7 représente une zone active et des connexions électriques incorporées dans le dispositif présenté en figure 1.

La figure 8 représente des polariseurs pouvant s'incorporer dans le dispositif présenté en figure 1.

Les figures 9, 10, 11 et 12 représentent des schémas-blocs de circuits électroniques incorporés dans le dispositif présenté en figure 1.

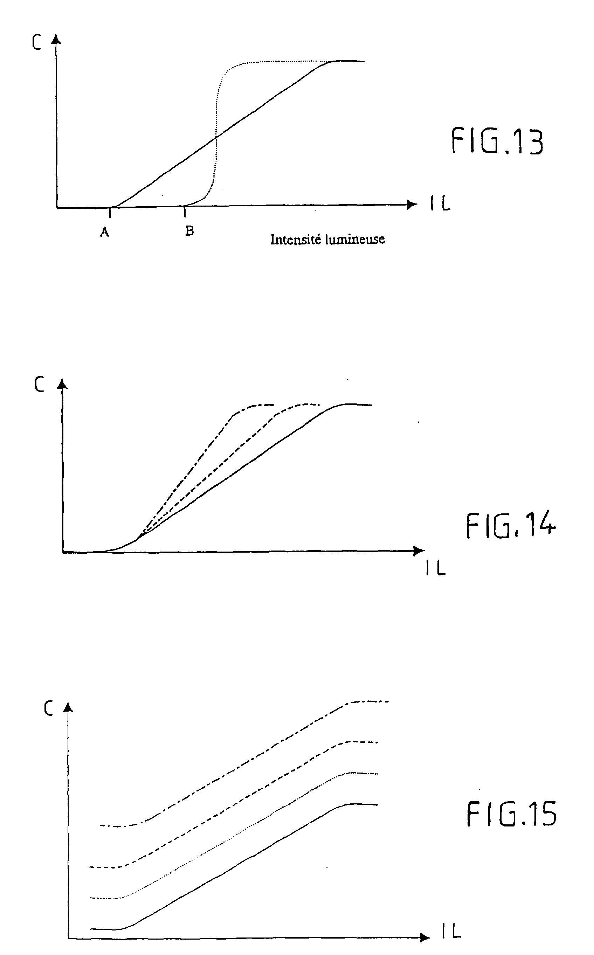

Ces schémas permettent la régulation des filtres électro-optiques des systèmes cités dont les réponses désirées sont décrites figures 13, 14 et 15.

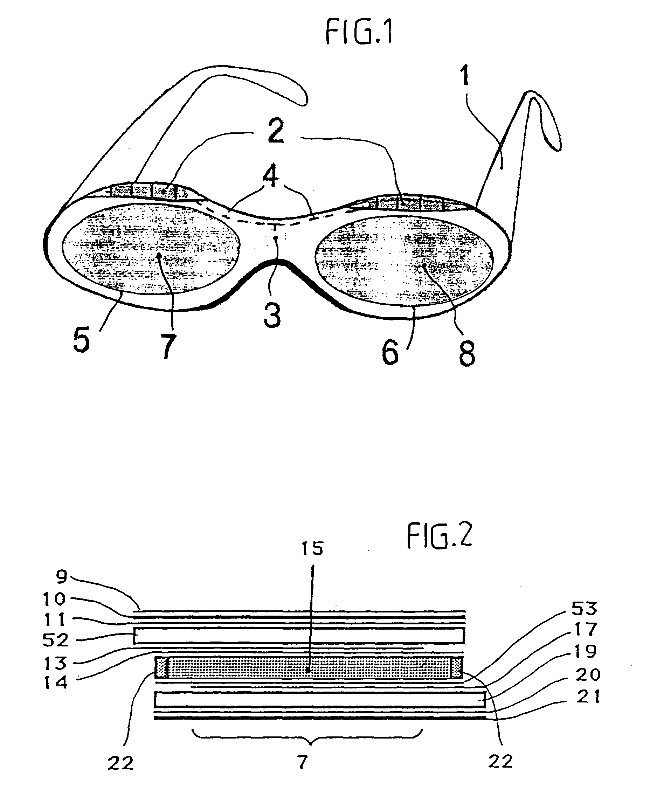

Dans la figure 1 sont représentés une monture de lunetterie (1), un capteur photo-sensible (2), un circuit électronique (3), deux filtres électro-optiques (5) et (6) comportant des zones actives (7) et (8) respectivement, et des connexions électriques (4) reliant le circuit électronique (3) et les zones actives (7) et (8) des filtres électro-optiques (5) et (6).
La monture de lunetterie (1) est de type connu. Elle est adaptée pour supporter mécaniquement les différents composants précités. En particulier, la monture (1) peut être réalisée par moulage ou par usinage de pièces en plastique, en métal ou en matériaux synthétiques.

Le capteur photo-sensible (2)est adapté à émettre un signal continu dont la puissance est une fonction croissante de l'intensité lumineuse totale qui lui parvient. Par exemple, le capteur photosensible (2) peut être constitué d'une photo-diode, d'une photo-résistance alimentée par une pile électrique ou une cellule solaire.

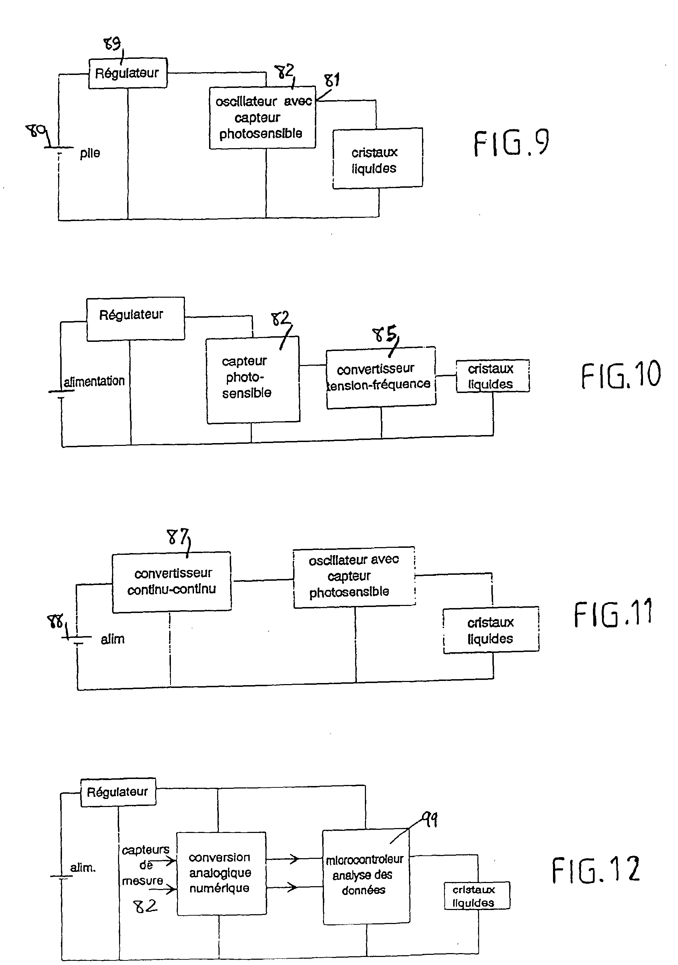

Le circuit électronique (3) est relié électriquement au capteur photosensible (2) et est apte à émettre un signal alternatif de puissance croissante avec la puissance du signal reçu du capteur photosensible (2). Un exemple de circuit électronique (3) est donné en figures 9 à 12.

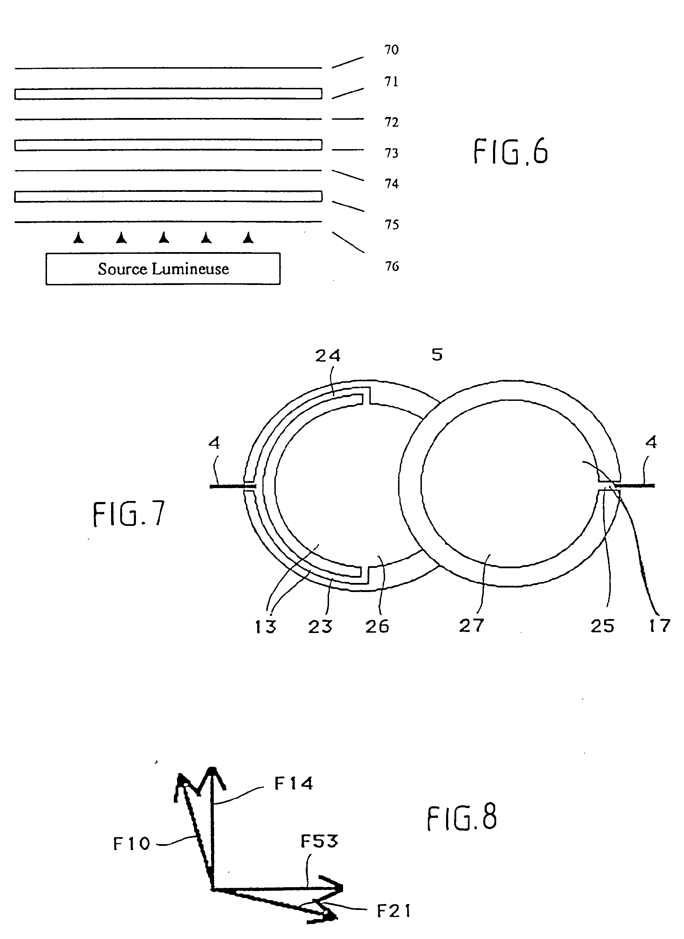

Les filtres électro-optiques (5) et (6) fonctionnent en positif, c'est-à-dire que leur transparence diminue avec la puissance du signal électrique qui leur est appliquée. Les filtres électro-optiques (5) et (6) comportent respectivement les zones actives (7) et (8) présentées en figure 7. Ces zones actives (7) et (8) sont positionnées devant les yeux de l'utilisateur du dispositif dans le cas où le dispositif est une paire de lunettes. Les zones actives (7) et (8) sont connexes au sens mathématique du terme, c'est-à-dire que tous les segments de droite reliant deux points quelconques de chaque zone active sont intégralement dans la zone active.
Les liaisons électriques (4) sont présentées en figure 7. Elles relient électriquement le circuit électronique (3) et chacun des filtres electro-optiques (5) et (6).

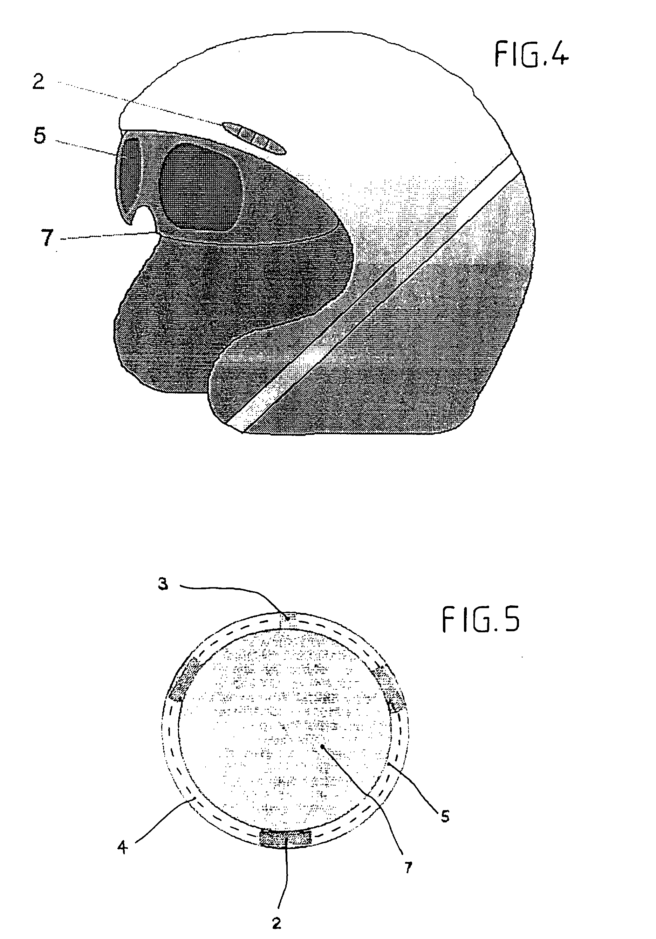

La figure 2 représente une vue en coupe d'un des écrans electro-optiques incorporés dans le dispositif représenté en figure 1 et le principe de base des applications citées figures 4 et 5.

On peut distinguer, toutefois, deux catégories d'application des filtres électro-optiques, utilisant le principe de base illustré en figure 2. La première catégorie correspond à la protection contre les rayons lumineux. On retrouve ainsi concernés les filtres pour lunettes, jumelles, caméras, appareils photographiques, rétroviseurs, pare-soleil. La seconde catégorie vise à améliorer la luminosité d'ambiance, le confort et l'esthétique. Parmi les applications impliquées, on peut citer les vitrages d'habitation ou de bureau, les lumières d'ambiance (lampadaires), les phares de véhicules automobiles. Dans ce but, on peut procéder à un mélange de filtres de couleurs différentes dont le schéma figure 4 décrit la conception.

Dans la figure 2 sont représentés successivement, de haut en bas, un traitement anti-rayure (9), un polariseur (10), une couche de colle (11), une lame de verre (52), un électrode (13), une couche d'orientation (53), une électrode (14), un cristal liquide (15), une couche d'orientation (53), une électrode (17),une lame de verre (19), une couche de colle (20), un polariseur (21), une zone active (7) et, latéralement positionnés autour du cristal liquide (15) et entre les couches d'orientation (14) et (53), un ruban de colle (22).
On retrouve de même, dans la solution de la figure 6, un empilage verre (70,72,74,76) « film de cristaux liquides » (71,73,75) que l'on peut associer à des polariseurs.

Selon une autre caractéristique de l'invention, chacun des filtres peut être commandé indépendamment ou de façon synchrone par l'électronique qui lui est attribuée.

Le traitement anti-rayure (9) est réalisé sur le polariseur (10) de manière connue. Les polariseurs (10) et (21) sont de type connu. Il sont collés sur les lames de verre (52) et (19) par les couches de colle (11) et (20). Les électrodes (13) et (17) sont transparentes et de type connu et réalisées sur les lames de verre ( 52) et (19). Les couches d'orientation (14) et (53) et le ruban de colle (22) sont de type connu dans la réalisation d'écrans à cristaux liquides. Le cristal liquide (15) est de type nématique en hélice avec une hélice réalisant une portion de son pas suivant le degré d'obscurcissement souhaité. Cet angle peut varier de 60 à 90°. On privilégie le lus souvent des valeurs approchant les 90° pour un plus fort obscurcissement. Les orientations des polariseurs et des couches d'orientation sont présentées en figure 8.

La zone active connexe (7) est la partie connexe la plus grande de la superposition des électrodes (13) et (17).

La figure 7 représente une zone active et des connexions électriques incorporées dans le dispositif présenté en figure 1.

Dans la figure 7, sont représentés le filtre électro-optique (5), comportant les électrodes (13) et (17), la zone active (7) entre des parties d'électrodes (26) et (27) et des segments d'électrodes (23,24,25) et les liaisons électriques (4). L'électrode (13) est constituée des segments (23) et (24) d'une part, et de la partie d'électrode (26) d'autre part. L'électrode (17) est constituée du segments (25) d'une part, et de la partie d'électrode (27) d'autre part. Les parties d'électrodes (26) et (27) se superposent exactement et sont identiques à la zone active connexe (7).

La figure 8 représente des polariseurs pouvant s'incorporer dans le dispositif présenté en figure 1 et autres exemples d'applications.

Dans la figure 8, sont représentés quatre vecteurs : F10, F14, F53 et F21. Le vecteur F10 représente la direction de polarisation du polariseur (10). Le vecteur F14 représente la direction d'orientation de la couche d'orientation (14). Le vecteur F53) représente la direction d'orientation de la couche d'orientation (53). Le vecteur F21 représente la direction de polarisation du polariseur (21).

Les vecteurs F14 et F53 sont perpendiculaires, ce qui est connu dans la fabrication des écrans à cristaux liquides nématiques en hélice. Le vecteur F10 est incliné vers la gauche et possède avec le vecteur F14 un angle de cinq degrés dans le sens trigonométrique. La flèche F21 est incliné vers le bas et possède avec la flèche F53 un angle de cinq degrés dans le sens opposé au sens trigonométrique. L'angle entre les directions de polarisation des polariseurs (10) et (21) est donc de cent degrés. Il est à noter que d'autres valeurs d'inclinaison par rapport aux directions des couches d'orientation peuvent être envisagés et que les valeurs des angles entre les couches d'orientation et les polariseurs peuvent être différentes ou dans le même sens trigonométrique. Préférentiellement, les écrans électro-optiques (5) et (6) sont constitués d'écrans à cristaux liquides comportant des couches d'orientation (14) et (53) et des polariseurs (10) et (21). Au moins un angle entre l'une des directions F14 ou F53 d'une couche d'orientation et respectivement l'une des directions F10 ou F21 d'un polariseur est supérieur à deux degrés.

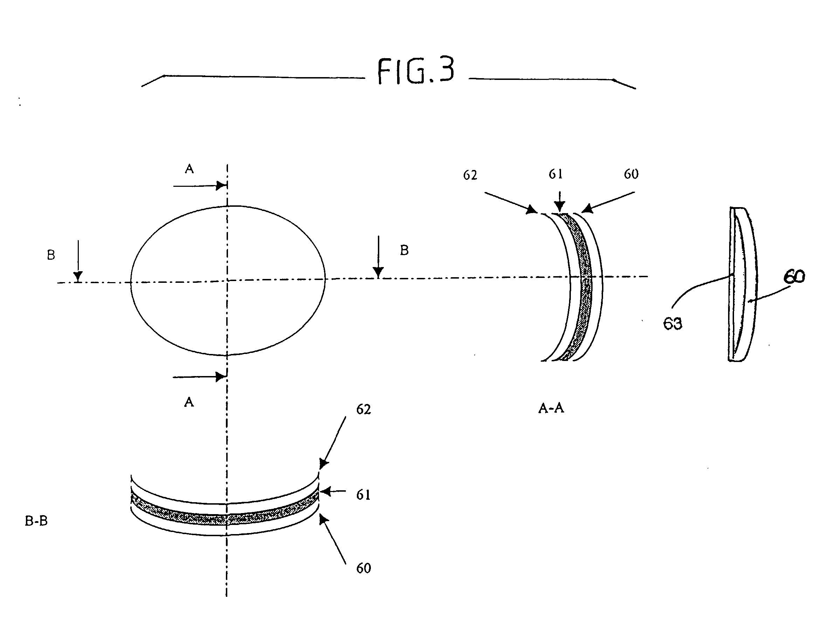

Selon une autre caractéristique de l'invention, le filtre électro-optique présente une courbure de manière à être associé à un système correcteur. Ainsi, le filtre électro-optique à cristaux liquides peut présenter une courbure suivant deux directions imposées par des verres bombés (voir figure 3). Des matériaux, tels le polycarbonate ou dérivés, des polymères, peuvent être utilisés en remplacement du verre pour leurs propriétés de mise en oeuvre.

Le filtre électro-optique courbe peut avoir comme caractéristique une structure interne fonctionnelle s'apparentant à celle de la figure 2 (référence 62), mais qui, souvent, peut aussi comporter un système optique.

En effet, un système optique permet une adaptation de l'invention aux « lunettes de vue » avec la correction optique adaptée à l'utilisateur.

Une première lentille permet d'obtenir (60) un chemin lumineux perpendiculaire aux cristaux liquides, la seconde (61) rétablissant l'image.

La courbure donnée aux verres est importante dans le cas de lunettes solaires ou de vue, non seulement pour une question d'esthétique mais aussi pour résoudre les effets gênants dits « rétroviseurs » que procurent les verres plats.

En outre, on peut adjoindre un traitement anti-reflets pour pallier à cet inconvénient.

Une solution de verres courbes (60) associés à un dispositif conforme à la figure 2 mais de faible épaisseur (63) permet d'obtenir la courbure extérieure et un dispositif de filtre plan indépendamment.

De même, des traitements anti UV et infra-rouges doivent être apportés aux verres afin de protéger l'utilisateur dans le spectre visible et ultra violet.

Sur les figures 9, 10, 11 et 12, sont représentés les schémas-blocs électroniques des filtres électro-optiques. On retrouve dans toutes les configurations une alimentation (80), un capteur photosensible (81)et un oscillateur (82).

L'alimentation peut être constituée à partir d'une pile ou d'une batterie, de cellules solaires photo-voltaîques ou être incorporée dans le capteur photosensible.

Comme le montre la figure 13 (courbe 83), la réponse en tension donnée par des capteurs solaires n'est pas linéaire en fonction du flux lumineux reçu. Le circuit électronique ne fonctionnant qu'en faisant varier l'amplitude du signal appliqué au filtre électro-optique, n'est totalement effectif qu'à partir d'un certain seuil. La régulation en dessous de celui-ci est impossible alors qu'au dessus, on atteint vite la saturation (voir figure 13).

Pour résoudre ce problème, le circuit électrique courbé (83) est apte à faire varier la fréquence du signal secondaire, l'augmentation de l'intensité lumineuse se traduisant par une réduction de la fréquence pour donner un résultat illustré à la figure 13, courbe (84).

Selon une première forme de réalisation de l'invention, le circuit électrique comporte un oscillateur asservi en fréquence en fonction de l'intensité lumineuse (figure 10).

Sur la figure 10, on a ainsi représenté l'oscillateur (85) alimenté par le capteur photosensible (82).

Selon une autre forme de réalisation, on peut agir à la fois sur la tension et la fréquence puisqu'une augmentation brutale de la tension peut être compensée par une augmentation de la fréquence de l'oscillateur. En effet, les deux paramètres agissent en sens inverse sur le contraste produit par les cristaux liquides. Dans ce cas, l'oscillateur est également asservi en amplitude, l'augmentation de l'intensité se traduisant par une plus grande amplitude.

La bande passante en fréquence des cristaux liquides étant assez large (50 Hz à 3000 Hz), elle permet, outre un fonctionnement de l'oscillateur à tension constante, la possibilité de régler facilement le niveau de contraste (figure 14). (chaque niveau correspondant à une fréquence d'oscillation).
En effet, suivant le type de verre et d'utilisateur, on peut agir sur l'asservissement électronique afin de fixer la réponse désirée. Il s'agit, par exemple, de la solution proposée figure 11, combinant tension et fréquence, et figure 12 par un microprocesseur réalisant sans difficulté la linéarité et le niveau du contraste.

Avantageusement, l'utilisation de cellules photovoltaïques ou de type photodiode permet non seulement l'autonomie énergétique et délivrent une tension significative pour un flux lumineux minimal.

L'oscillateur nécessite une tension supérieure au volt pour être déclenché. La très faible consommation des cristaux liquides (quelques micro-ampères), permet, pour la seule utilisation d'un convertisseur continu-continu (87) d'élever la tension délivrée par les cellules (88). De ce fait, le nombre de cellules se trouve réduit et le seuil du début de fonctionnement est bas (figure 13) (plage de régulation très large à partir du point A au lieu de B).

Dans le cas de la figure 9, on utilise un régulateur (89) qui servira de stabilisateur de tension. Le capteur photosensible (82) commandera l'oscillateur (81) en tension afin que celui-ci délivre un signal alternatif d'amplitude variable de façon proportionnelle et linéaire à la luminosité, l'action sur la fréquence pouvant être associée pour obtenir différents niveaux de contraste (figure 15).

Par ailleurs, on a pu constater qu'une augmentation d'amplitude ou une diminution de fréquence du signal d'alimentation des filtres électro-optiques permettait un assombrissement de ces derniers de façon proportionnelle. Il est donc nécessaire comme le montre la figure 10 que le capteur photosensible agisse sur l'amplitude ou la fréquence de l'oscillateur, ce qui est réalisable avec certains capteurs photosensibles. Ces derniers présentent une variation de résistance en fonction de la luminosité, ce qui permet de faire varier la tension ou la fréquence par la constante de temps de l'oscillateur. En effet, celle-ci dépend toujours de la résistance et du condensateur nécessaire à l'oscillateur. La figure 9 constitue une variante intéressante dans le cas d'une alimentation par cellules solaires photovoltaïques. En effet, la tension délivrée par celle-ci est souvent faible lorsque leur nombre est réduit et ne permet pas le fonctionnement de l'oscillateur avec peu de luminosité. C'est pourquoi l'apport d'un convertisseur continu-continu peut être intéressant pour élever la tension compte tenu de la faible puissance nécessaire à l'oscillateur.

La figure 12 illustre une solution à base de micro-contrôleur (99) pouvant tenir compte d'autres paramètres que la luminosité, en vue d'assurer la variation de la fréquence. Le micro-contrôleur est interfacé avec le capteur de mesure (82) par un convertisseur analogique numérique type connu et approprié.

Selon une forme de réalisation avantageuse, le micro-contrôleur est apte à assurer également la variation d'amplitude du signal.

Le microprocesseur, grâce à son programme interne, fabrique le signal variable de commande adapté au filtre après avoir pris en compte tous les paramètres déjà cités.

Il est entendu que ces systèmes pourront être mis en oeuvre avec différentes technologies de type C.M.S. (Composants Montés en Surface) ou hybride ou encore la construction d'un circuit spécialisé de type A.S.I.C.

Afin d'obtenir plus de contraste et de puissance dans certaines applications, on pourra associer à l'oscillateur un amplificateur électronique dont le gain pourra éventuellement varier.

De même, dans certains cas, tels les vitrages d'habitation ou lumières d'ambiance, l'électronique de fonctionnement décrite par les schémas des figures 9 à 12 pourra être complétée par une commande à distance de type infrarouge ou hyper fréquence.

Cette électronique de type classique « émetteur-récepteur » permet de faire croître ou décroître, par impulsions successives ou par potentiomètre, les paramètres tension ou fréquence de l'oscillateur de façon à faire varier le contraste du filtre. Plusieurs filtres peuvent être mis en oeuvre par cette méthode, chacun pouvant, par exemple, avoir un code de différenciation.

Préférentiellement, l'axe de polarisation des polariseurs avant de chacun des écrans électro-optiques (5) et (6) est vertical.

## Revendications

1. Dispositif optique à filtre électro-optique comportant :
• un capteur photosensible,
• un circuit électronique électriquement relié au capteur photosensible,
• et au moins un écran électro-optique fonctionnant en positif et comprenant une zone active et reliée par des liaisons électriques à la sortie du circuit électronique,
**caractérisé :**
- en ce que le capteur photo-sensible est adapté à émettre un signal de puissance croissante, en fonction de l'intensité lumineuse qui l'atteint,
- et en ce que le circuit électronique, d'une part, émet un signal secondaire alternatif de puissance croissante avec la puissance du signal émis par le capteur photo-sensible et, d'autre part, présente des moyens aptes à faire varier la fréquence du signal secondaire, l'augmentation de l'intensité lumineuse se traduisant par une réduction de la fréquence .

2. Dispositif optique selon la revendication 1, caractérisé en ce que les moyens sont constitués par un oscillateur asservi en fréquence en fonction de ladite intensité lumineuse.

3. Dispositif optique selon la revendication 2, caractérisé en ce que l'oscillateur est également asservi en amplitude, l'augmentation de l'intensité lumineuse se traduisant par une augmentation de l'amplitude.

4. Dispositif optique selon la revendication 1, caractérisé en ce que les moyens sont constitués par un microcontrôleur apte à assurer la variation de la fréquence.

5. Dispositif optique selon la revendication 4, caractérisé en ce que le microcontrôleur est apte à assurer la variation d'amplitude du signal.

6. Dispositif optique selon l'une des revendications 4 ou 5, caractérisé en ce que le microcontrôleur est associé à une commande à distance.

7. Dispositif optique selon l'une des revendications précédentes caractérisé en ce que le circuit électronique est alimenté par au moins une cellule photovoltaïque.

8. Dispositif optique selon la revendication 7, caractérisé en ce que le circuit électronique comprend un convertisseur continu intercalé entre la cellule photovoltaïque et l'oscillateur.

9. Dispositif optique selon l'une des revendications précédentes, caractérisé en ce que le filtre électro-optique présente une courbure de manière à être associé à un système correcteur.

10. Dispositif optique selon l'une des revendications précédentes, caractérisé en ce qu'il présente plusieurs filtres optiques commandés indépendamment et de façon synchrone.
